(19) **Europäisches Patentamt**
**European Patent Office**
**Office européen des brevets**

(11) **EP 4 692 268 A1**

(12) **EUROPEAN PATENT APPLICATION**
published in accordance with Art. 153(4) EPC

(43) Date of publication:
**11.02.2026 Bulletin 2026/07**

(21) Application number: **24784776.7**

(22) Date of filing: **26.03.2024**

(51) International Patent Classification (IPC):
*C09J 175/08* (2006.01)    *C09J 7/38* (2018.01)
*C09J 11/06* (2006.01)    *A61F 13/02* (2024.01)
*A61F 13/0246* (2024.01)    *C08G 18/10* (2006.01)
*C08G 18/48* (2006.01)

(52) Cooperative Patent Classification (CPC):
**C09J 7/38; C09J 11/06; C09J 175/08;** A61F 13/02;
A61F 13/0246; C08G 18/10; C08G 18/48

(86) International application number:
**PCT/JP2024/011799**

(87) International publication number:
**WO 2024/209986 (10.10.2024 Gazette 2024/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.04.2023 JP 2023061902**
**13.07.2023 JP 2023115052**
**03.10.2023 JP 2023172078**

(71) Applicant: **AGC INC.**
**Chiyoda-ku,**
**Tokyo 1008405 (JP)**

(72) Inventors:
• **KOMINE Takuya**
**Tokyo 100-8405 (JP)**
• **SASAZAKI Hiroki**
**Tokyo 100-8405 (JP)**
• **NAKAMURA Makito**
**Tokyo 100-8405 (JP)**

(74) Representative: **Müller-Boré & Partner**
**Patentanwälte PartG mbB**
**Friedenheimer Brücke 21**
**80639 München (DE)**

(54) **ADHESIVE COMPOSITION, PRODUCTION METHOD THEREFOR, ADHESIVE, PATCH, WEARABLE DEVICE, AND WEARABLE DEVICE KIT**

(57)   Provided are an adhesive composition that can provide an adhesive agent that has a good balance between the adhesive force to the skin and the adhesive force to a device, is skin friendly (capable of gently attaching and detaching an adherend to and from the skin, and low toxic), and has improved antibacterial properties, and the like. An adhesive composition comprising a hydroxyl-terminated urethane prepolymer and a polyisocyanate compound, in which the hydroxyl-terminated urethane prepolymer is a reaction product of an oxyalkylene polymer and a diisocyanate compound in the presence of a tin-free catalyst, the oxyalkylene polymer includes an oxyalkylene polymer A having the number of hydroxyl groups per molecule of 2 or more and an oxyalkylene polymer B having the number of hydroxyl groups per molecule of 1, and the hydroxyl-terminated urethane prepolymer has an average content of structural units based on ethylene oxide of 8 to 50% by mass.

EP 4 692 268 A1

## Description

Technical Field

The present invention relates to an adhesive composition to obtain an adhesive agent applicable to paste on the skin, a method for producing the same, an adhesive agent, a patch, a wearable device, and a wearable device kit.

Background Art

Adhesive agents for skin patches, such as adhesive plaster, surgical tapes, poultices, and plaster agents, and adhesive agents used for pasting a patch-type wearable device on the skin to continuously obtain biometrics such as body temperature and electroencephalogram have been employed.

As the adhesive agents, for example, PTL1 describes an acrylic-based adhesive agent that is not applied on the skin, but contains an organoiodine-based living radical polymerization initiator and has improved antibacterial properties due to the inclusion of iodine.

Further, PTL2 describes an acrylic-based adhesive agent that is a non-sensitizing pressure sensitive adhesive agent that is useful for medical applications and other uses in contact with skin, and selected so that a cured adhesive agent passes an in vitro cytotoxicity test in accordance with ISO 10993-5:2009.

Citation List

Patent Literature

PTL 1: JP 2022-98300 A

PTL 2: JP 2022-541292 A

Summary of Invention

Technical Problem

However, an adhesive agent that has a good balance between the adhesive force to the skin and the adhesive force to a device, is skin friendly (capable of gently attaching and detaching an adherend to and from the skin, and low toxic), and has improved antibacterial properties has not yet been obtained, whereby there has been a strong desire to develop such an adhesive agent.

The present invention has been made in consideration of these circumstances, and aims to provide an adhesive composition that can provide an adhesive agent that has a good balance between the adhesive force to the skin and the adhesive force to a device, is skin friendly (capable of gently attaching and detaching an adherend to and from the skin, and low toxic), and has improved antibacterial properties, a method for producing the same, as well as an adhesive agent, a patch, a wearable device, and a wearable device kit which are obtained by using the adhesive composition.

Solution to Problem

The present invention is based on the discovery of a polyurethane-based adhesive agent which has a good balance between the adhesive force to the skin and the adhesive force to a device, without using a tin-based catalyst, the use of which is gradually being restricted from the viewpoint of the impact on health, and the like.

The present invention provides the following means.

[1] An adhesive composition comprising a hydroxyl-terminated urethane prepolymer and a polyisocyanate compound, in which the hydroxyl-terminated urethane prepolymer is a reaction product of an oxyalkylene polymer and a diisocyanate compound in the presence of a tin-free catalyst, the oxyalkylene polymer includes an oxyalkylene polymer A having the number of hydroxyl groups per molecule of 2 or more and an oxyalkylene polymer B having the number of hydroxyl groups per molecule of 1, and the hydroxyl-terminated urethane prepolymer has an average content of structural units based on ethylene oxide of 8 to 50% by mass.

[2] The adhesive composition according to [1], in which the content of the tin-free catalyst is more than 0.001 parts by mass and less than 0.050 parts by mass per 100 parts by mass of the oxyalkylene polymer.

[3] The adhesive composition according to [1] or [2], in which the tin-free catalyst is an organometallic catalyst.

[4] The adhesive composition according to any of [1] to [3], in which the tin-free catalyst comprises one or more metals selected from the group consisting of zinc and bismuth.

[5] The adhesive composition according to any of [1] to [4], in which the oxyalkylene polymer has an average content of structural units based on ethylene oxide of 8 to 50% by mass.

[6] The adhesive composition according to any of [1] to [5], in which the oxyalkylene polymer has an average number of hydroxyl groups per molecule of 1.5 to 3.0.

[7] The adhesive composition according to any of [1] to [6], in which the oxyalkylene polymer A comprises an oxyalkylene polymer A1 having the number of hydroxyl groups per molecule of 3 and an oxyalkylene polymer A2 having the number of hydroxyl groups per molecule of 2.

[8] The adhesive composition according to any of [1] to [7], in which the oxyalkylene polymer A has an average content of structural units based on ethylene oxide of 0 to 80% by mass.

[9] The adhesive composition according to any of [1] to [8], in which the number-average molecular weight of the oxyalkylene polymer B is 4000 to 30000.

[10] The adhesive composition according to any of [1] to [9], used for forming an adhesive agent that comes into contact with skin.

[11] An adhesive composition comprising a hydroxyl-terminated urethane prepolymer, a polyisocyanate compound, and a tin-free catalyst, in which the hydroxyl-terminated urethane prepolymer has an average content of structural units based on ethylene oxide of 8 to 50% by mass.

[12] A method for producing an adhesive composition by reacting an oxyalkylene polymer and a diisocyanate compound in the presence of a tin-free catalyst to obtain a hydroxyl-terminated urethane prepolymer, and mixing the hydroxyl-terminated urethane prepolymer and a polyisocyanate compound, in which the oxyalkylene polymer includes an oxyalkylene polymer A having the number of hydroxyl groups per molecule of 2 or more and an oxyalkylene polymer B having the number of hydroxyl groups per molecule of 1, and the hydroxyl-terminated urethane prepolymer has an average content of structural units based on ethylene oxide of 8 to 50% by mass.

[13] An adhesive agent, being a cured product of the adhesive composition according to any of [1] to [11].

[14] The adhesive agent according to [13], which passes the antibacterial test standard described in ISO22196:2011 or JIS Z2801:2010.

[15] A patch comprising a substrate and an adhesive agent layer provided on at least a part of a surface of the substrate, in which the adhesive agent layer comprises the adhesive agent according to [13] or [14].

[16] The patch according to [15], being an adhesive tape.

[17] A wearable device comprising a wearable device main body and an adhesive agent layer, in which the adhesive agent layer is provided on at least a part of the surface of the wearable device facing the skin and comprises the adhesive agent according to [13] or [14].

[18] A wearable device kit comprising a wearable device main body, and at least any of the adhesive agent according to [13] or [14] and the patch according to [15] or [16].

Advantageous Effects of Invention

According to the present invention, composition that can provide an adhesive agent that has a good balance between the adhesive force to the skin and the adhesive force to a device, is skin friendly (capable of gently attaching and detaching an adherend to and from the skin, and low toxic), and has improved antibacterial properties, and a method for producing the same.

Also, according to the present invention, provided are an adhesive agent and a patch using the same, as well as a wearable device and a wearable device kit.

Description of Embodiments

The definitions and meanings of the terms and notations described herein are shown in the followings.

The skin refers to human skin.

The number-average molecular weight (Mn) and weight-average molecular weight (Mw) are polystyrene equivalent molecular weights determined by measuring with gel permeation chromatography (GPC) based on calibration curves created by using standard polystyrene samples.

The hydroxyl value is determined by measurement in accordance with JIS K 1557-1:2007.

The degree of unsaturation is determined by measurement in accordance with JIS K 1557-3:2007.

The colony formation rate is determined by a cytotoxicity test using a colony formation method by an extract method, in accordance with ISO 10993-5:2009 "Biological evaluation of medical devices -- Part 5: Tests for in vitro cytotoxicity". Specifically, it can be determined by the methods described in Examples.

The isocyanate index indicates an equivalent ratio of isocyanate groups to be reacted and active hydrogen-containing groups (e.g., hydroxyl group) ([isocyanate group]/[active hydrogen-containing groups]), expressed as percentage.

[Adhesive Composition]

The adhesive composition in an embodiment of the present invention (hereinafter, referred to as the present embodiment) contains a hydroxyl-terminated urethane prepolymer and a polyisocyanate compound, the hydroxyl-terminated urethane prepolymer is a reaction product of an oxyalkylene polymer and a diisocyanate compound in the presence of a tin-free catalyst, the oxyalkylene polymer includes an oxyalkylene polymer A having the number of hydroxyl groups per molecule of 2 or more and an oxyalkylene polymer B having the number of hydroxyl groups per molecule of 1, and the hydroxyl-terminated urethane prepolymer has an average content of structural units based on ethylene oxide of 8 to 50% by mass.

Using an adhesive composition using a hydroxyl-terminated urethane prepolymer generated by a reaction of a specified oxyalkylene polymer and a diisocyanate compound in the presence of a tin-free catalyst and having an average content of structural units based on ethylene oxide within a predetermined range, it is possible to obtain an adhesive agent having a good balance between the adhesive force to the skin and the adhesive force to a device and being skin friendly (capable of gently attaching and detaching an adherend to and from the skin, and low toxic).

The adhesive composition in the present embodiment can be used for forming an adhesive agent to be in contact with the skin.

The average content of structural units based on ethylene oxide (hereinafter, abbreviated as "EO units") in the adhesive composition is not particularly limited and is preferably 8 to 50% by mass, more preferably 8 to 30% by mass or less, further preferably 8 to 20% by mass, yet further preferably 8 to 13% by mass, and particularly preferably 8 to 11% by mass, from the viewpoint of obtaining an adhesive agent having a good balance between the adhesive force to the skin and the adhesive force to a device, being skin friendly (capable of gently attaching and detaching an adherend to and from the skin, and low toxic), and having improved antibacterial properties.

The average content of EO units in the adhesive composition is derived from EO units constituting oxyalkylene chains of the oxyalkylene polymer in the hydroxyl-terminated urethane prepolymer, and the content can be determined by analyzing the monomer composition of the oxyalkylene chains in the adhesive composition through measurement of $^{13}$C-NMR (nuclear magnetic resonance) in the adhesive composition.

(Hydroxyl-Terminated Urethane Prepolymer)

The hydroxyl-terminated urethane prepolymer of the present embodiment is a reaction product of an oxyalkylene polymer and a diisocyanate compound in the presence of a tin-free catalyst.

The average content of EO units in the hydroxyl-terminated urethane prepolymer is not particularly limited as long as it falls within 8 to 50% by mass, and is preferably 8 to 30% by mass, more preferably 8 to 20% by mass, further preferably 8 to 13% by mass, and particularly preferably 8 to 11% by mass.

The lower the average content of EO units in the hydroxyl-terminated urethane prepolymer, the lower the toxicity to the skin, and when the average content falls within the above range, it is easy to obtain an adhesive agent that can gently attach and detach an adherend to and from the skin, has a good balance between the adhesive force to the skin and the adhesive force to a device, and has improved antibacterial properties.

The EO units in the hydroxyl-terminated urethane prepolymer are derived from EO units constituting the oxyalkylene chains of the oxyalkylene polymer, and the average content can be determined by analyzing the monomer composition of the oxyalkylene chains in the hydroxyl-terminated urethane prepolymer through measurement of $^{13}$C-NMR (nuclear magnetic resonance).

<Oxyalkylene Polymer>

The oxyalkylene polymer comprises an oxyalkylene polymer A having the number of hydroxyl groups per molecule of 2 or more (hereinafter, also simply referred to as "polymer A") and an oxyalkylene polymer B having the number of hydroxyl groups per molecule of 1 (hereinafter, also simply referred to as "polymer B").

It is preferable that the hydroxyl groups of the polymer A and the polymer B react with the isocyanate group of the diisocyanate compound to form a urethane bond, and unreacted hydroxyl groups correspond to terminal hydroxyl groups of the molecular chain of the hydroxyl-terminated urethane prepolymer.

The average content of EO units in the oxyalkylene polymer is not particularly limited, and is preferably 8 to 50% by mass, more preferably 8 to 30% by mass, further preferably 8 to 20% by mass, yet further preferably 8 to 13% by mass, and particularly preferably 8 to 11% by mass.

The average content of EO units in the oxyalkylene polymer means the weight-averaged value of the contents of EO units in each oxyalkylene polymer based on the amounts of a plurality of the oxyalkylene polymers blended used in the synthesis of the hydroxyl-terminated urethane prepolymer. The content of EO units in each of the oxyalkylene polymers is a value calculated based on the amount of EO blended in the raw material in the synthesis of the oxyalkylene polymers. Note that the content of EO units in the oxyalkylene polymer can also be determined through measurement of $^{13}$C-NMR, in the same manner as the content of EO units in the hydroxyl-terminated urethane prepolymer.

The lower the average content of EO units in the oxyalkylene polymer, the lower the toxicity to the skin, and when the average content falls within the above range, it is easy to obtain an adhesive agent that can gently attach and detach an adherend to and from the skin, has a good balance between the adhesive force to the skin and the adhesive force to a device, and has improved antibacterial properties.

The oxyalkylene polymer has an average number of hydroxyl groups per molecule of preferably 1.5 to 3.0, more preferably 1.7 to 2.8, and further preferably 1.9 to 2.6, from the viewpoint of obtaining an adhesive agent being skin friendly (capable of gently attaching and detaching an adherend to and from the skin, and low toxic) and having a good balance between the adhesive force to the skin and the adhesive force to a device. The average number of hydroxyl groups per molecule of the oxyalkylene polymer means the weight-averaged value based on the amounts of a plurality of the oxyalkylene polymers blended used in the synthesis of the hydroxyl-terminated urethane prepolymer.

The oxyalkylene polymer may be one oxyalkylene polymer, or two or more oxyalkylene polymers.

The total content of the polymer A and the polymer B in the oxyalkylene polymer is preferably 80% by mass or more, more preferably 90% by mass or more, further preferably 95% by mass or more, and yet further preferably 100% by mass.

The content ratio of the polymer A and the polymer B in the oxyalkylene polymer is preferably 10/90 to 95/5, more preferably 30/70 to 90/10, further preferably 50/50 to 90/10, and yet further preferably 60/40 to 90/10 at a mass ratio (polymer A/polymer B), from the viewpoint of obtaining an adhesive agent having a good balance between the adhesive force to the skin and the adhesive force to a device.

<<Oxyalkylene Polymer A>>

The number of hydroxyl groups per molecule of the polymer A is 2 or more, and preferably 2 to 6.

The polymer A may be one oxyalkylene polymer, or two or more oxyalkylene polymers. When the polymer A consists of two or more oxyalkylene polymers, good adhesive force of the adhesive agent is easily adjusted.

When the polymer A consists of two or more oxyalkylene polymers, the average number of hydroxyl groups per molecule of the polymer A is preferably 2.1 to 3.0, more preferably 2.2 to 2.9, and further preferably 2.3 to 2.8.

When the average number of hydroxyl groups of the polymer A falls within the above range, it is easy to obtain an adhesive agent that suppresses a load on the skin where a device is fixed, and is less likely to leave adhesive residues on the skin.

The average number of hydroxyl groups per molecule of the polymer A can be calculated by the hydroxyl value of the polymer A and the measured value of Mn, using the formula of the hydroxyl value $\times$ Mn/56100.

When the polymer A consists of two or more oxyalkylene polymers, examples of the polymer A include an oxyalkylene polymer A1 having the number of hydroxyl groups per molecule of 3 (hereinafter, also simply referred to as "polymer A1"), and an oxyalkylene polymer A2 having the number of hydroxyl groups per molecule of 2 (hereinafter, also simply referred to as "polymer A2"). One polymer A1 may be used alone, or two or more may be used. Similarly, one polymer A2 may be used alone, or two or more may be used.

The polymer A can contain an oxyalkylene polymer having the number of hydroxyl groups per molecule of 4.

Note that when the polymer A consists of the polymer A1 and the polymer A2, the weight-averaged value of the number of hydroxyl groups per molecule of 3 of the polymer A1 and the number of hydroxyl groups per molecule of 2 of the polymer A2 based on the composition ratio (amount blended) of the polymer A1 and the polymer A2 can be considered as the average number of hydroxyl groups of the polymer A.

For example, the average number of hydroxyl groups per molecule of the polymer A in example 1 (Synthesis Example 1) described later can be calculated by the following formula (1).

$$(3 \times 0.36/8470 + 2 \times 0.48/9510)/(0.36/8470 + 0.48/9510) = 2.5 \text{ ... Formula (1)}$$

Out of a total of 100 parts by mass of the polymer A, the total amount of the polymer A1 and the polymer A2 accounts for preferably 85 parts by mass or more, more preferably 90 parts by mass or more, and further preferably 95 parts by mass or more, from the viewpoint of ease of adjustment and the like, in which the adhesive force of the adhesive agent can be adjusted by adjusting the ratio of the polymer A1 and the polymer A2.

Out of a total of 100 parts by mass of the polymer A, it is particularly preferably that the total amount of the polymer A1 and the polymer A2 account for 100 parts by mass, that is, the polymer A consist of the polymer A1 and the polymer A2

From the viewpoint of good adhesive force, suppression of adhesive residues, and the like of the adhesive agent, the polymer A1 accounts for preferably 20 parts by mass or more, more preferably 25 to 95 parts by mass, and further preferably 30 to 90 parts by mass, out of a total of 100 parts by mass of the polymer A1 and the polymer A2.

From the similar viewpoint, the polymer A2 accounts for preferably 80 parts by mass or less, more preferably 5 to 75 parts by mass, and further preferably 10 to 70 parts by mass, out of a total of 100 parts by mass of the polymer A1 and the polymer A2.

When the polymer A consists of the polymer A1 and the polymer A2, the polymer A2 accounts for preferably 10 parts by mass or more, more preferably 10 to 300 parts by mass, and further preferably 50 to 200 parts by mass per 100 parts by mass of the polymer A1, from the viewpoint of good adhesive force, suppression of adhesive residues, and the like of the adhesive agent.

When the polymer A consists of, for example, the polymer A1 and the polymer A2, the content of the polymer A1 accounts for preferably 10 to 45 parts by mass, more preferably 15 to 40 parts by mass, and further preferably 30 to 40 parts by mass, out of a total of 100 parts by mass of the polymer A1, the polymer A2, and the polymer B in the oxyalkylene polymer, from the viewpoint of obtaining an adhesive agent having a good balance between the adhesive force to the skin and the adhesive force to a device.

From the similar viewpoint, the content of the polymer A2 accounts for preferably 15 to 50 parts by mass, more preferably 30 to 50 parts by mass, and further preferably 40 to 50 parts by mass, out of a total of 100 parts by mass of the polymer A1, the polymer A2, and the polymer B in the oxyalkylene polymer.

From the similar viewpoint, the content of the polymer B accounts for preferably 5 to 40 parts by mass, more preferably 10 to 35 parts by mass, and further preferably 10 to 20 parts by mass, out of a total of 100 parts by mass of the polymer A1, the polymer A2, and the polymer B in the oxyalkylene polymer.

The content ratio of the polymer A1, the polymer A2, and the polymer B is preferably the polymer A2, the polymer A1, and the polymer B in descending order.

The average content of EO units in the polymer A is preferably 0 to 80% by mass, more preferably 0 to 70% by mass, further preferably 0 to 60% by mass, and yet further preferably 0 to 50% by mass.

The average content of EO units in the polymer A can be determined by analyzing the monomer composition of oxyalkylene chains in the polymer A through the measurement of $^{13}$C-NMR. For example, when the oxyalkylene chains in the polymer A consist of EO units and PO units, the content of EO units can be calculated based on the area of the peak showing methylene groups of the EO units and the area of the peak showing methyl groups of the PO units.

When the polymer A consists of, for example, the polymer A1 and the polymer A2, the same applies to the respective content of EO units in the polymer A1 and the polymer A2.

Note that the average content of EO units in the polymer A can be considered as a value obtained by calculating from the amount of EO blended in a raw material for synthesis of the polymer A. When the polymer A consists of, for example, the polymer A1 and the polymer A2, the weight-averaged value of the content of EO units of the polymer A1 and the content of EO units of the polymer A2 based on the composition ratio (amount blended) of the polymer A1 and the polymer A2 can be considered as the average content of EO units of the polymer A.

The polymer A has Mn of preferably 1000 to 50000, more preferably 5000 to 30000, and further preferably 8000 to 25000, from the viewpoint of good adhesive force, suppression of adhesive residues, good flexibility, and the like of the adhesive agent.

Mw/Mn (molecular weight distribution) of the polymer A is closed to 1, and preferably 1.25 or less, more preferably 1.22 or less, and further preferably 1.20 or less, since the narrower the molecular weight distribution, the less likely the hydroxyl-terminated urethane prepolymer becomes high viscous, leading to increased synthesis efficiency.

In addition, the degree of unsaturation of the polymer A is preferable to be closer to 0 meq/g, preferably 0.020 meq/g or less, more preferably 0.018 meq/g or less, and further preferably 0.015 meq/g or less, from the viewpoint of good curability of the hydroxyl-terminated urethane prepolymer, suppression of adhesive residues of the adhesive agent, and the like.

When the polymer A consists of two or more oxyalkylene polymers, each of the oxyalkylene polymers preferably falls within the above Mn, Mw/Mn, and degree of unsaturation.

The synthesis method of the polymer A is not particularly limited, and for example, the polymer A can be obtained by ring-opening addition polymerization of a compound having a cyclic ether structure, preferably alkylene oxide, to an initiator having two or more active hydrogens in the presence of a catalyst. When the polymer A consists of the polymer A1 and the polymer A2, a method can be used in which the ring-opening addition polymerization of a compound having a cyclic ether structure is carried out by concurrently using an initiator having three active hydrogens and an initiator having two active hydrogens to simultaneously generate the polymer A1 and the polymer A2. From the viewpoint of adjusting the amount of the polymer A1 blended and the polymer A2 more precisely, a method is preferable in which the polymer A1 obtained by the ring-opening addition polymerization of a compound having a cyclic ether structure to an initiator having three active hydrogens and the polymer A2 obtained by the ring-opening addition polymerization of a compound having a cyclic ether structure to an initiator having two active hydrogens are separately synthesized and then mixed together.

The compound having a cyclic ether structure is preferably linear or branched having 2 to 14 carbon atoms, more preferably 2 to 10 carbon atoms, and further preferably 2 to 4 carbon atoms.

The compound having a cyclic ether structure may be used alone, or in combination of two or more.

Examples of the compound having a cyclic ether structure include ethylene oxide (EO), propylene oxide (PO), 1,2-butylene oxide, 2,3-butylene oxide, methyl glycidyl ether, butyl glycidyl ether, 2-ethylhexyl glycidyl ether, lauryl glycidyl ether, hexyl glycidyl ether, and tetrahydrofuran. Among these, EO and PO are preferable.

When two or more compounds having a cyclic ether structure are used in combination, for the polymer A, the sequence of oxyalkylene groups derived from each of the compounds may be random or blocked.

Examples of the group having an active hydrogen in the initiator include hydroxyl groups, carboxy groups, and amino groups having a hydrogen atom bound to a nitrogen atom. Among these, the hydroxyl group is preferable, and the alcoholic hydroxyl group is more preferable.

Examples of the initiator having three hydroxyl groups include glycerin, trimethylolethane, trimethylolpropane, and 1,2,6-hexanetriol. Any one of these may be used alone, or two or more may be used in combination.

Examples of the initiator having two hydroxyl groups include ethylene glycol, diethylene glycol, propylene glycol, and dipropylene glycol. Any one of these may be used alone, or two or more may be used in combination.

The number of active hydrogens of the initiator generally corresponds to the number of hydroxyl groups per molecule of the oxyalkylene polymer. The polymer A1 can be synthesized, for example, by using glycerin as the initiator. Also, the polymer A2 can be synthesized, for example, by using propylene glycol as the initiator.

Note that the type and amount of the initiator used as a raw material for synthesis of the oxyalkylene polymer can be identified through the measurement of $^{13}$C-NMR. When the polymer A consists of the polymer A1 and the polymer A2, the ratio of the polymer A1 to the polymer A2 is determined by determining the content ratio of the oxyalkylene groups, such as the EO units and PO units, bound to each initiator, from the information on the type and amount of the initiator for the polymer A through the measurement of $^{13}$C-NMR.

The ring-opening addition polymerization can be performed by using a known catalyst, for example, alkali catalysts such as potassium hydroxide, transition metal compoundporphyrin complex catalysts such as a complex obtained by reacting an organic aluminum compound with porphyrin, double metal cyanide complex catalysts, and catalysts consisting of a phosphazene compound. Among these catalysts, the double metal cyanide complex (DMC) catalyst is preferable, since an oxyalkylene polymer having narrow molecular weight distribution and relatively low viscosity is easily obtained. As the double metal cyanide complex include, a known compound can be used, and examples thereof include a zinc hexacyanocobaltate complex with tert-butanol as the ligand.

The synthesis of the oxyalkylene polymer by the ring-opening addition polymerization using a DMC catalyst can be performed by a known method, and for example, a production method described in WO 2003/062301, WO 2004/067633, JP 2004-269776 A, JP 2005-15786 A, WO 2013-065802, and JP 2015-10162 A can be applied.

A commercially available product can be used as the polymer A. Examples of the commercially available product as the polymer A include "Preminol (registered trademark; hereinafter omitted) S 4013F", "Preminol S 4318F", "Preminol S 3011", "Preminol 5001F", "Preminol 7001K", "Preminol 7012", "Preminol S 4011", "Preminol S 4015", "Preminol S 3025", and "Preminol S 6420" (all manufactured by AGC Inc.); "ACCLAIM (registered trademark; hereinafter omitted) 4200", "ACCLAIM 8200", "ACCLAIM 2220N", "ACCLAIM 3300N", "ACCLAIM 4220N", "ACCLAIM 6300", "Acclaim 2200", and "ACCLAIM 6320N" (all manufactured by Covestro AG).

<<Oxyalkylene Polymer B>>

The number of hydroxyl groups per molecule of the oxyalkylene polymer B is 1. The polymer B may be one oxyalkylene polymer, or two or more oxyalkylene polymers.

The number of hydroxyl groups of the polymer B can be confirmed by identifying the type and amount of the initiator used as a raw material through the measurement of $^{13}$C-NMR as in the case of the polymer A.

The polymer B has Mn of preferably 4000 to 30000, more preferably 4500 to 25000, and further preferably 5000 to 20000, from the viewpoint of formation of an adhesive agent layer having good flexibility, good adhesive force, suppression of adhesive residues, and the like of the adhesive agent.

Mw/Mn of the polymer B is closed to 1, and preferably less than 1.20, more preferably less than 1.13, and further preferably less than 1.10, since the narrower the molecular weight distribution, the less likely the hydroxyl-terminated urethane prepolymer becomes high viscous, leading to increased synthesis efficiency.

In addition, the degree of unsaturation of the polymer B is preferably 0.015 meq/g or less, more preferably 0.013 meq/g or less, and further preferably 0.010 meq/g or less, and it is preferable to be closer to 0 meq/g, from the viewpoint of good curability of the hydroxyl-terminated urethane prepolymer, suppression of adhesive residues of the adhesive agent layer, and the like.

When the polymer B consists of two or more oxyalkylene polymers, each of the oxyalkylene polymers preferably falls within the above Mn, Mw/Mn, and degree of unsaturation.

The synthesis method of the polymer B is not particularly limited, and for example, the polymer B can be obtained by ring-opening addition polymerization of a compound having a cyclic ether structure, preferably alkylene oxide, to an initiator having one active hydrogen in the presence of a catalyst.

The compound having a cyclic ether structure is similar to those described for the polymer A. PO alone, or EO and PO in combination is preferable.

The polymer B preferably contains an average content of EO units of 0 to 80% by mass, more preferably 0 to 60% by mass, and further preferably 0 to 50% by mass, from the viewpoint of obtaining an adhesive agent having a good balance between the adhesive force to the skin and the adhesive force to a device, and its crystallinity being suppressed.

The average content of EO units in the polymer B can be determined as in the case of the polymer A.

The group having an active hydrogen in the initiator includes those similar to the case of the polymer A, and the hydroxyl group is preferable, and the alcoholic hydroxyl group is more preferable.

The initiator having one hydroxyl group is preferably a monohydric alcohol having 2 to 4 carbon atoms, from the viewpoint of ease of availability, and the like; examples thereof include n-propanol, isopanol, n-butanol, sec-butanol, isobutanol, and tert-butanol, and among these, n-butanol, sec-butanol, isobutanol, and tert-butanol are preferable. One initiator may be used alone, or two or more may be used in combination.

The ring-opening addition polymerization can be performed by a known method as in the case of the polymer A.

A commercially available product can be used as the polymer B. Examples of the commercially available product as the polymer B include "Preminol S 1011", "Preminol S 1004F" (manufactured by AGC Inc.).

<Diisocyanate Compound>

The diisocyanate compound used for synthesis of the hydroxyl-terminated urethane prepolymer is an organic compound having 2 isocyanate groups in a molecule. The diisocyanate compound may be an aliphatic diisocyanate compound, an alicyclic diisocyanate compound, an aromatic diisocyanate compound, or an aromatic aliphatic diisocyanate compound. One diisocyanate compound may be used alone, or two or more may be used in combination.

An aliphatic diisocyanate compound may be either linear or branched, and examples thereof include tetramethylene diisocyanate, hexamethylene diisocyanate (HDI), dodecamethylene diisocyanate, 2,2,4-trimethylhexamethylene diisocyanate, 2,4,4-trimethylhexamethylene diisocyanate, lysine diisocyanate, 2-methylpentane-1,5-diisocyanate, and 3-methylpentane-1,5-diisocyanate.

As an alicyclic diisocyanate compound, examples thereof include isophorone diisocyanate (IPDI), hydrogenated xylylene diisocyanate, 4,4'-dicyclohexylmethane diisocyanate (HMDI), 1,4-cyclohexane diisocyanate, methylcyclohexylene diisocyanate, and 1,3-bis(isocyanatomethyl)cyclohexane.

As an aromatic diisocyanate compound, examples thereof include tolylene diisocyanate, 2,2'-diphenylmethane diisocyanate, 2,4'-diphenylmethane diisocyanate, 4,4'-diphenylmethane diisocyanate (MDI), 4,4'-dibenzyl diisocyanate, 1,5-naphthylene diisocyanate, 1,3-phenylene diisocyanate, 1,4-phenylene diisocyanate, dialkyl diphenylmethane diisocyanate, and tetraalkyl diphenylmethane diisocyanate.

As an aromatic aliphatic diisocyanate compound, examples thereof include xylylene diisocyanate, and tetramethylxylylene diisocyanate (TMXDI).

Among these, HDI, IPDI, HMDI, and TMXDI are preferable, and HDI is more preferable from the viewpoint of obtaining an adhesive agent layer having good curability and good flexibility of the hydroxyl-terminated urethane prepolymer.

Furthermore, as the diisocyanate compound, a bi-functional isocyanate-terminated urethane prepolymer obtained by previously reacting the diisocyanate compound as described above with diol (e.g., ethylene glycol, propylene glycol, 1,4-butanediol, 1,5-pentanediol, 3-methyl-1,5-pentanediol, and 1,6-hexanediol) can be used.

<Tin-Free Catalyst>

The tin-free catalyst is an urethanization catalyst that does not contain tin. In the present embodiment, any tin-containing catalyst is not used as the urethanization catalyst.

The adhesive agent of the present embodiment does not contain tin and a tin compound from the viewpoint of obtaining an adhesive agent being low toxic and capable of gently attaching and detaching an adherend to and from the skin.

Examples of the tin-free catalyst include tertiary amine catalysts, and organometallic catalysts containing metals other than tin. One catalyst may be used alone, or two or more may be used in combination. The tin-free catalyst is preferably a catalyst containing metals other than tin from the viewpoint of better reaction promoting effects.

Examples of metals contained in the organometallic catalyst containing metals other than tin include zinc, bismuth, titanium, lead, iron, cobalt, and zirconium. Among these, zinc and bismuth are preferable, and zinc is more preferable from the viewpoint of low toxicity to the skin, ease of handling, and the like. The catalyst containing metals other than tin is preferably an organometallic catalyst containing metals other than tin from the viewpoint of better reaction promoting effects. Examples of metals contained in the organometallic catalyst are the same as the above-mentioned metals contained in the catalyst containing metals other than tin, and preferred metals are also the same.

Examples of the tertiary amine catalyst include triethylamine, triethylenediamine, and 1,8-diazabicyclo[5.4.0]-7-undecene (DBU).

Examples of the organometallic catalyst containing metals other than tin include zinc compounds such as zinc carboxylates, for example, zinc naphthenate and zinc 2-ethylhexanoate, and zinc acetylacetonate; bismuth compounds such as bismuth 2-ethylhexanoate and bismuth neodecanoate; titanium compounds such as dibutyltitanium dichloride, tetrabutyltitanate, and butoxytitanium trichloride; lead compounds such as lead oleate, lead 2-ethylhexanoate, lead benzoate, and lead naphthenate; iron compounds such as iron 2-ethylhexanoate, iron acetylacetonate; cobalt compounds such as cobalt benzoate and cobalt 2-ethylhexanoate; and zirconium compounds such as zirconium naphthenate.

The amount of the tin-free catalyst used in the reaction of an oxyalkylene polymer and a diisocyanate compound is preferably more than 0.001 parts by mass and less than 0.050 parts by mass, more preferably 0.005 to 0.045 parts by mass, and particularly preferably 0.010 to 0.040 parts by mass per 100 parts by mass of the oxyalkylene polymer, from the viewpoint of good reaction promoting effects and reduction of amount of residues of metal components.

(Polyisocyanate Compound)

The polyisocyanate compound is a curing agent for the hydroxyl-terminated urethane prepolymer.

The polyisocyanate compound is a compound having two or more isocyanate groups in a molecule; and one polyisocyanate compound may be used alone, or two or more may be used in combination.

The polyisocyanate compound is preferably a diisocyanate compound, from the viewpoint of ease of availability, reactivity, and the like. In addition, the polyisocyanate compound having 3 or more isocyanate groups in a molecule is preferable, from the viewpoint of good adhesive force, suppression of adhesive residues, and the like of the adhesive agent.

The amount of polyisocyanate compound added is not particularly limited, and is preferably 1 to 40 parts by mass, more preferably 2 to 30 parts by mass, and particularly preferably 5 to 20 parts by mass, based on a total of 100 parts by mass of the hydroxyl-terminated urethane prepolymer.

Specific examples of the diisocyanate compound include those similar to the specific examples of the diisocyanate compound constituting the hydroxyl-terminated urethane prepolymer, as described above.

Examples of the polyisocyanate compound having 3 or more isocyanate groups in a molecule include isocyanurate modified products, biuret modified products, allophanate modified products, and tri-functional or higher isocyanate-terminated urethane prepolymers (adducts) which are reaction products of a diisocyanate compound and polyol having 3 or more hydroxyl groups in one molecule (e.g., trimethylolpropane), all of which are derivatives of the diisocyanate compound described above.

(Other Components)

The adhesive composition may contain other components other than the hydroxyl-terminated urethane prepolymer and the polyisocyanate compound. Examples of the other components include various additives such as a plasticizer, an antioxidant, an antistatic agent, a filler, an ultraviolet absorber, a light stabilizer, a conductivity imparting agent, and a leveling agent. Also, a solvent may be contained. The other components may be used alone, or two or more may be used in combination; and these can be blended at a content within a range that does not impair the effects of the present invention.

The total content of the hydroxyl-terminated urethane prepolymer and the polyisocyanate compound in the adhesive composition (excluding the solvent) is preferably 85% by mass or more, more preferably 90% by mass or more, further preferably 95% by mass or more, and yet further preferably 100% by mass from the viewpoint of sufficiently exerting the effects of the present invention.

The plasticizer can be selected from the viewpoint of compatibility with other components contained, wettability of the adhesive agent layer to an adherend, and the like, and examples thereof include fatty acid ester and phosphoric acid ester, that have 8 to 30 carbon atoms.

The antioxidant contributes to the suppression of thermal deterioration of the adhesive agent layer, or the like; examples thereof include phenol-based, amine-based, sulfur-based, and phosphorous-based antioxidants, and among these, the phenol-based antioxidant is preferable from the viewpoint of low toxicity to the skin.

The antistatic agent contributes to the suppression of damage to the circuit pattern due to electrostatic discharge, and examples thereof include inorganic salts; ionic liquids containing imidazolium ions, pyridinium ions, ammonium ions, or the like; and surfactants.

Examples of the filler include talc, calcium carbonate, and titanium oxide.

Examples of the ultraviolet absorber include benzophenone-based, benzotriazolebased, salicylic acid-based, oxalic acid anilide-based, cyanoacrylate-based, and triazine-based ultraviolet absorbers.

Examples of the light stabilizer include hindered amine-based light stabilizers and ultraviolet stabilizers.

Examples of the conductivity imparting agent include metal microparticles such as silver nanoparticles.

Examples of the leveling agent include polymer-based leveling agents such as acrylic-based, vinyl-based, silicone-based, and fluorine-based leveling agent.

The adhesive composition in one embodiment of the present invention is an adhesive composition containing a hydroxyl-terminated urethane prepolymer, a polyisocyanate compound, and a tin-free catalyst, and the hydroxyl-terminated urethane prepolymer has an average content of structural units based on ethylene oxide of 8 to 50% by mass.

The hydroxyl-terminated urethane prepolymer, the polyisocyanate compound, and the tin-free catalyst in the adhesive composition are preferably the same as the hydroxyl-terminated urethane prepolymer, the polyisocyanate compound, and the tin-free catalyst described above, respectively. Other components other than the hydroxyl-terminated urethane prepolymer, the polyisocyanate compound, and the tin-free catalyst are also the same as the other components described above.

[Method for Producing Adhesive Composition]

The method for producing an adhesive composition of the present embodiment is a method for producing an adhesive composition by reacting an oxyalkylene polymer and a diisocyanate compound in the presence of a tin-free catalyst to obtain a hydroxyl-terminated urethane prepolymer, and mixing the hydroxyl-terminated urethane prepolymer and a polyisocyanate compound, in which the oxyalkylene polymer includes an oxyalkylene polymer A having the number of hydroxyl groups per molecule of 2 or more and an oxyalkylene polymer B having the number of hydroxyl groups per molecule of 1, and the hydroxyl-terminated urethane prepolymer has an average content of structural units based on ethylene oxide of 8 to 50% by mass.

Note that, when the hydroxyl-terminated urethane prepolymer and the polyisocyanate compound are mixed, the other components that can be contained in the adhesive composition described above may also be mixed.

The hydroxyl-terminated urethane prepolymer can be obtained by reacting an oxyalkylene polymer and a diisocyanate compound in the presence of a tin-free catalyst. The oxyalkylene polymer, the diisocyanate compound, and the tin-free catalyst are the same as those described above.

The hydroxyl-terminated urethane prepolymer can be produced, for example, by a method in which the oxyalkylene polymer, the diisocyanate compound, a catalyst and a solvent are all charged at once in a reaction vessel, or a method in which the diisocyanate compound is subsequently added dropwise or the like in a reaction vessel where the oxyalkylene polymer, a catalyst and a solvent have been charged. The method of subsequently adding the diisocyanate compound is preferable, from the viewpoint of ease of molecular weight distribution control of the hydroxyl-terminated urethane prepolymer due to preferential reaction of low-molecular components in the raw material for synthesis.

Examples of the solvent include ketons such as acetone, and methylethylketon; esters such as ethyl acetate; and aromatic hydrocarbons such as toluene, and xylene. One solvent may be used alone, or two or more may be used in combination.

When using a solvent, the amount thereof used is preferably 50 to 500 parts by mass, more preferably 70 to 400 parts by mass, and further preferably 80 to 300 parts by mass, based on a total of 100 parts by mass of the oxyalkylene polymer, from the viewpoint of uniformity of the reaction systems and synthesis efficiency.

It is preferable that the oxyalkylene polymer and the diisocyanate compound be reacted at the isocyanate index of 100 or less, preferably 30 to 95, and more preferably 50 to 95, from the viewpoint of efficiently obtaining a hydroxyl-terminated urethane prepolymer having an appropriate molecular chain length.

The reaction temperature is preferably less than 100°C, more preferably 70 to 95°C, and further preferably 75 to 90°C, from the viewpoint of promoting the urethanization reaction and suppressing the side reaction.

After completion of the reaction, a terminator for reaction such as acetylacetone may be added thereto in order to quench the catalyst.

The average number of hydroxyl groups per molecule of the hydroxyl-terminated urethane prepolymer is preferably 3.0 or less, more preferably 1.7 to 2.9, and further preferably 1.8 to 2.5, from the viewpoint of obtaining an adhesive agent having a good balance between the adhesive force to the skin and the adhesive force to a device.

The isocyanate index of the polyisocyanate compound used to react with the hydroxyl-terminated urethane prepolymer is preferably more than 100, more preferably 101 to 1500, and further preferably 105 to 1000.

[Adhesive Agent]

The adhesive agent of the present embodiment is a cured product of the adhesive composition of the present embodiment described above.

An adhesive agent can be obtained as a reaction product of the hydroxyl-terminated urethane prepolymer and the polyisocyanate compound in the adhesive composition. As described later, an adhesive agent can be formed as an adhesive agent layer by, for example, applying and curing the adhesive composition on a substrate.

For the reaction of the hydroxyl-terminated urethane prepolymer and the polyisocyanate compound, a catalyst and a solvent can be used as necessary.

When using a catalyst, a urethanization catalyst, the tin-free catalyst described above, can be used. The catalyst may be same as or different from those used in the synthesis of the hydroxyl-terminated urethane prepolymer. When the catalyst used in the synthesis of the hydroxyl-terminated urethane prepolymer remains, the remaining catalyst can also act as a catalyst in the reaction of the hydroxyl-terminated urethane prepolymer and the polyisocyanate compound.

When the catalyst is added in the reaction of the hydroxyl-terminated urethane prepolymer and the polyisocyanate compound, the amount to be added is more than 0.001 parts by mass and less than 0.050 parts by mass, preferably 0.005 to 0.045 parts by mass, and more preferably 0.010 to 0.040 parts by mass, based on a total of 100 parts by mass of the hydroxyl-terminated urethane prepolymer and the polyisocyanate compound. When the catalyst is added, after completion of the reaction, it is preferable to add a reaction terminator in order to quench the catalyst.

Examples of the solvent used in the reaction of the hydroxyl-terminated urethane prepolymer and the polyisocyanate compound include those similar to the case of the above-mentioned hydroxyl-terminated urethane prepolymer. The catalyst may be same as or different from those used in the synthesis of the hydroxyl-terminated urethane prepolymer. When the solvent used in the synthesis of the hydroxyl-terminated urethane prepolymer remains, it can be used as it is.

When using a solvent, the amount thereof used is preferably 50 to 500 parts by mass, more preferably 70 to 400 parts by mass, and further preferably 80 to 300 parts by mass, based on a total of 100 parts by mass of the hydroxyl-terminated urethane prepolymer and the polyisocyanate compound.

The reaction temperature between the hydroxyl-terminated urethane prepolymer and the polyisocyanate compound is preferably less than 120°C, more preferably 40 to 110°C, and further preferably 50 to 100°C, from the viewpoint of promoting the urethanization reaction and suppressing the side reaction.

The adhesive agent of the present embodiment is preferably low toxic when applied to the skin, and the colony formation rate in a cytotoxicity test using the colony formation method specified in ISO 10993-5:2009 is preferably 50% or more, and more preferably 70% or more.

It is preferable for the adhesive agent of the present embodiment to pass the antibacterial test standard described in ISO22196:2011 or JIS Z2801:2010.

[Patch]

The patch of the present embodiment has a substrate and an adhesive agent layer provided on at least a part of the surface of the substrate, and the adhesive agent layer contains the above-mentioned adhesive agent of the present embodiment.

The patch having the adhesive agent layer formed by using the adhesive composition of the present embodiment has a good adhesive force to the skin of the adhesive agent layer, is less likely to leave adhesive residues, and can be appropriately applied to the skin.

The form of the patch may be a desired sheet shape of a size according to the usage mode, or may be cut to a desired size for use.

The patch is preferably an adhesive tape from the viewpoint of convenience.

The adhesive composition used to form an adhesive agent layer may be a onecomponent type in which a hydroxyl-terminated urethane prepolymer and a polyisocyanate compound, which is a curing agent, are mixed in advance, or a two-component type consisting of a first component that contains a hydroxyl-terminated urethane prepolymer and a second component that contains a polyisocyanate compound, which is a curing agent, depending on the usage mode of the patch, or the like.

The thickness of the adhesive agent layer is preferably 5 to 100 $\mu$m, more preferably 10 to 80 $\mu$m, and further preferably 20 to 50 $\mu$m, from the viewpoint of securing good adhesive force on the skin.

The substrate is preferably made of a material that has good followability of the patch to the shapes of the skin or a device, and that is less likely to be broken when the patch is used, and examples thereof include those made of a resin film, fabric, paper, or the like. The substrate may be used alone, or two or more may be used in combination, and it may also be a composite material by laminating layers or the like.

Examples of the material for the resin film include polyurethane; polyamide; acrylic resin; polyolefins such as polyethylene, polypropylene, and ethylene-vinyl acetate copolymer; and polyester. Among these, polyurethane and polyamide are preferable from the viewpoint of good moisture permeability when applied to the skin, and thermoplastic polyurethane is more preferable.

Examples of the fabric include woven fabric, non-woven fabric, knitted fabric, and net, and the properties of the material may be a resin, which is a material for the above-mentioned resin film, and natural fibers such as cotton, silk, and wool.

The thickness of the substrate is not particularly limited as long as the followability to the shapes of the skin or the device is secured, and the substrate is not easily broken when the patch is used, and examples thereof are 10 to 500 μm.

The surface of the adhesive agent layer in the patch is preferably covered with a release liner for the protection of the surface of the adhesive agent layer before using the patch. The release liner is appropriately peeled off from the surface of the adhesive agent layer when the patch is used.

A known release liner used for adhesive tapes for skin can be applied to the release liner. Examples of the release liner include woodfree paper, glassine paper, or parchment paper on the surface of which a release agent such as a silicone resin or fluororesin is coated, and woodfree paper anchor coated or polyethylene laminated with resin, on the surface of which a release agent such as a silicone resin or fluororesin is coated. In addition, as the release liner, a resin film having transparency can be used, and examples thereof include a polyester film.

The thickness of the release liner is not particularly limited as long as it can protect the adhesive agent layer and it can be easily peeled when used, and examples thereof are 15 to 200 μm.

The adhesive agent layer may be provided, for example, on a part of the surface of the substrate made of a resin film at one or two or more locations, may be provided on only a part of one side, or may be provided on the entire surface of one side. Also, it may be provided on both sides of the surface of the substrate.

When the adhesive force of the adhesive agent layer to the skin is preferably 1.0 to 5.0 N/15 mm, and more preferably 1.0 N/15 mm or more and less than 3.0 N/15 mm, the adhesive agent layer can be said to have an adequate adhesive force to the skin.

Also, when the adhesive agent layer is adhered to a sealant of a device, or the like, for example, the adhesive force to a phenol resin, which is common material as the sealant, is preferably greater than the adhesive force to the skin. The adhesive force to the phenol resin is preferably 2.5 N/15 mm or more, and more preferably 3.0 N/15 mm or more, from the viewpoint of sufficiently fixing a device.

The difference between the adhesive force to the phenol resin and the adhesive force to the skin is preferably 1.8 N/15 mm or more, and more preferably 2.5 N/15 mm or more. When there is such a difference, it can be said that the balance between the adhesive force to the skin and the adhesive force to the device is good, and the load on the skin to which the device is fixed is low.

Note that the adhesive force used herein is a value measured by a method in accordance with JIS Z 0237:2009, and specifically, it is measured by the methods described in Examples.

The method for producing a patch can be performed using a known method, and examples thereof include a method of applying and curing an adhesive composition on a release liner to form an adhesive agent layer, and adhering the substrate thereon. Another example is a method of applying and curing an adhesive composition on the substrate to form an adhesive agent layer, and adhering the release liner thereon.

The method of applying the adhesive composition is not particularly limited, and for example, known methods such as bar coding, knife coating, roll coating, blade coating, die coating, microgravure coating, comma coating, slot die coating, lip coating, and cast coating can be applied.

After application, adhesive composition is preferably cured by, for example, drying with hot air at 40 to 80°C to sufficiently volatilize the solvent contained in the adhesive composition, and thereby forming an adhesive agent layer.

[Wearable Device]

The wearable device of the present embodiment has a wearable device main body and an adhesive agent layer, and the adhesive agent layer is provided on at least a part of the surface of the wearable device facing the skin and contains the adhesive agent of the present embodiment.

When the above-mentioned adhesive composition of the present embodiment is used, an adhesive agent layer can be formed that has a good balance between the adhesive force to the skin and the adhesive force to the device and is skin friendly (capable of gently attaching and detaching an adherend to and from the skin, and low toxic). Therefore, the adhesive agent of the present embodiment is suitable for applying to a wearable device that is adhered to the skin.

The adhesive agent layer can be formed, for example, in the above-mentioned form of the patch.

Further, the adhesive agent layer may be formed in a manner of being sandwiched between release liners on both sides, and the wearable device main body and the skin may be adhered only via an adhesive agent layer, without using a substrate.

Examples of the wearable device pasted to the skin include a biosensor and environmental sensor. Examples of subjects detected by the biosensors include electrocardiography, pulse wave, body temperature, acceleration, heartbeat interval, pulse interval, respiration interval, electrocardiogram, electromyogram, activity level, blood pressure, and electroencephalogram.

[Wearable Device Kit]

The wearable device kit of the present embodiment contains a wearable device main body, and at least one of the adhesive agent of the present embodiment and the patch of the present embodiment In this way, the wearable device main body may be provided with an adhesive agent or a patch as another component, and comprised such that an adhesive agent can be formed when in use, not in the manner that the adhesive agent layer has in advice.

Either of the adhesive agent and patch may be used alone, or both may be used in combination.

Examples

Hereinafter, the present invention will be specifically described based on Examples; however, the present invention is not limited to the following Examples, and various modifications can be made without departing from the scope of the present invention.

[Measuring Methods]

Methods for measuring various physical properties in the following Synthesis Examples and Production Examples are as follows.

(Number-Average Molecular Weight (Mn) and Weight-Average Molecular Weight (Mw))

Mn and Mw were measured with gel permeation chromatography (GPC) in the following measurement conditions (in terms of polystyrene), the molecular weight distribution (Mw/Mn) was calculated from these values.

[Measurement Conditions]

- Apparatus used: "HLC-8320GPC", manufactured by Tosoh Corporation
- Column used: "TSKgel (registered trademark) SuperMultiporeHZ-M", manufactured by Tosoh Corporation
- Detector: Differential refractive index (RI) detector
- Detected temperature: 40°C
- Eluent: Tetrahydrofuran
- Flow rate: 0.350 mL/min
- Sample concentration: 0.5% by mass
- Amount of sample infused: 10 $\mu$L
- Standard sample: Polystyrene

(Content of Ethylene Oxide (EO) Units)

The content of EO units of each oxyalkylene polymer was based on the amount of propylene oxide (PO) and EO charged used for the synthesis, and the amount of EO charged was considered as the content of EO units in the oxyalkylene polymer.

The average content of EO units of the hydroxyl-terminated urethane prepolymer was designated as the weight-averaged value of the content of EO units in each component of the raw material.

(Hydroxyl Value)

The hydroxyl value was determined in accordance with B method (automatic potentiometric titration method) of JIS K 1557-1:2007.

(Degree of Unsaturation)

The degree of unsaturation was measured in accordance with JIS K 1557-3:2007.

(Average Number of Hydroxyl Groups Per Molecule of Oxyalkylene Polymer A)

The number of hydroxyl groups per molecule of the oxyalkylene polymers A1-1 to A2-2 used as the oxyalkylene polymer A (the number of hydroxyl groups per molecule of the initiator used for the synthesis) was weight-averaged based on each of the amounts of the oxyalkylene polymers A1-1 to A2-2 blended, and the obtained value was considered as the average number of hydroxyl groups per molecule.

(Average Number of Hydroxyl Groups Per Molecule of Oxyalkylene Polymer)

The number of hydroxyl groups per molecule of each of the oxyalkylene polymers (oxyalkylene polymers A1-1 to A2-2 and B1) used for the synthesis of the hydroxyl-terminated urethane prepolymer was weight-averaged based on each of the amounts of the oxyalkylene polymers blended, and the obtained value was considered as the average number of hydroxyl groups per molecule.

[Substances Used]

Substances used in the following Synthesis Examples are shown below in detail.

- TBA-DMC catalyst: zinc hexacyanocobaltate complex with tert-butanol as the ligand.
- Absorbent: Synthesized magnesium silicate; "KYOWAAD (registered trademark) 600S", manufactured by Kyowa Chemical Industry Co., Ltd.
- 50M-HDI: Hexamethylene diisocyanate (HDI); "Duranate (registered trademark) 50M-HDI", manufactured by Asahi Kasei Corporation
- XK627: "K-KAT XK627"; manufactured by KING INDUSTRIES, Inc.; zinc carboxylate: zinc (Zn-) containing urethanization catalyst
- Dibutyltin dilaurate; manufactured by Tokyo Chemical Industry Co., Ltd. ; Tin (Sn)-containing urethanization catalyst.
- Dioctyltin dilaurate; "NEOSTANE (registered trademark) U-810" manufactured by Nitto Kasei Co., Ltd. ; Sn-containing urethanization catalyst.
- Curing agent (polyisocyanate compound): "HDI-based polyisocyanate; Duranate (registered trademark) E402-80B", manufactured by Asahi Kasei Corporation; Solid content of about 80% by mass

[Synthesis of Oxyalkylene Polymer]

(Synthesis Example 1-1)

In a pressure-proof container, as the initiator, 1000 g of glycerin and TBA-DMC catalyst (metal concentration of 46 ppm by mass) was charged, and after nitrogen substitution in the container, the reaction solution was heated to 135°C while being stirred to react by adding 120g of propylene oxide (PO).

The reaction solution was cooled to 135°C after the temperature increase stopped, 3782.4 g of PO and 945.6 g of ethylene oxide (EO) were added into the container while stirring the reaction solution, and then after confirming that there was no more change in inner pressure, the absorbent was added thereto and neutralization and catalyst removal were performed to obtain oxyalkylene polymer A1-1.

(Synthesis Example 1-2)

Oxyalkylene polymer A1-2 was synthesized in the same manner as in Synthesis Example 1-1, except that no EO was added in Synthesis Example 1-1, and the amount of EO added was replaced with the amount of PO added.

(Synthesis Example 1-3)

Oxyalkylene polymer A2-1 was synthesized in the same manner as in Synthesis Example 1-1, except that the initiator in Synthesis Example 1-1 was changed from glycerin to propylene glycol.

(Synthesis Example 1-4)

Oxyalkylene polymer A2-2 was synthesized in the same manner as in Synthesis Example 1-2, except that the initiator in Synthesis Example 1-2 was changed from glycerin to propylene glycol, no EO was added, and the amount of EO added was replaced with the amount of PO added.

(Synthesis Example 1-5)

Oxyalkylene polymer B1 was synthesized in the same manner as in Synthesis Example 1-1, except that the initiator in Synthesis Example 1-1 was changed from glycerin to n-butanol, after the reaction solution was cooled, no EO was added but 2364 g of PO was added.

Various physical properties of the oxyalkylene polymers A1-1 to A2-2 and B1 produced in the above Synthesis Examples 1-1 to 1-5 are shown in the following Table 1.

[Table 1]

Table 1

| Synthesis examples | Polymer | The number of hydroxyl groups | The number-average molecular weight (Mn) | Degree of unsaturation [meq/g] | Hydroxyl value [mgKOH/g] | Molecular weight distribution (Mw/Mn) | The content of EO units [% by mass] |
|---|---|---|---|---|---|---|---|
| 1-1 | Polymer A1-1 | 3 | 8470 | 0.013 | 17.6 | 1.19 | 20 |
| 1-2 | Polymer A1-2 | 3 | 9090 | 0.005 | 16.9 | 1.10 | 0 |
| 1-3 | Polymer A2-1 | 2 | 9510 | 0.011 | 11.8 | 1.06 | 20 |
| 1-4 | Polymer A2-2 | 2 | 9990 | 0.006 | 10.7 | 1.06 | 0 |
| 1-5 | Polymer B1 | 1 | 5330 | 0.007 | 11.5 | 1.09 | 0 |

[Synthesis of Hydroxyl-Terminated Urethane Prepolymer]

(Synthesis Example 2-1)

In a reaction vessel equipped with a thermometer, a stirrer, and a cooling tube, oxyalkylene polymer A1-1 (36 parts by mass), oxyalkylene polymer A2-1 (48 parts by mass), oxyalkylene polymer B1 (16 parts by mass), 50.6 parts by mass of toluene and 50.6 parts by mass of ethyl acetate were charged, a zinc (Zn)-containing urethanization catalyst K-KAT XK627 (0.038 parts by mass) was added, and the mixture was mixed at 40°C, and then 1.7 parts by mass of HDI (isocyanate index of 85) was added thereto to react at 80°C.

The average number of hydroxyl groups per molecule of the blended oxyalkylene polymer A (oxyalkylene polymers A1-1 and A2-1) was 2.5, and the average number of hydroxyl groups per molecule of the oxyalkylene polymer (oxyalkylene polymers A1-1, A2-1, and B1) was 2.1.

While appropriately diluting the mixture with ethyl acetate, the mixture was maintained at 80°C and reacted for 7 hours to obtain a solution of transparent hydroxyl-terminated urethane prepolymer U1 with a concentration of 50% by mass.

(Synthesis Examples 2-2 to 2-11)

Hydroxyl-terminated urethane prepolymers U2 to U11 were prepared in the same manner as in Synthesis Example 1-1 using the blending compositions shown in Table 2.

[Table 2]

Table 2

| | | | Synthesis example 2-1 | Synthesis example 2-2 | Synthesis example 2-3 | Synthesis example 2-4 | Synthesis example 2-5 | Synthesis example 2-6 |
|---|---|---|---|---|---|---|---|---|
| Amount blended [part by mass] | Oxyalkylene polymer | Polymer A1-1 | 36 | 28 | 22 | 19 | 15 | 11 |
| | | Polymer A1-2 | - | 8 | 15 | 17 | 21 | 25 |
| | | Polymer A2-1 | 48 | 37 | 28 | 25 | 20 | 14 |
| | | Polymer A2-2 | - | 11 | 19 | 23 | 28 | 34 |
| | | Polymer B1 | 16 | 16 | 16 | 16 | 16 | 16 |
| | Diisocyanate compound | 50M-HDI | 1.7 | 1.7 | 1.7 | 1.7 | 1.7 | 1.7 |
| | Catalyst | XK627 | 0.038 | 0.038 | 0.038 | 0.038 | 0.038 | 0.038 |
| | | Dibutyltin dilaurate | - | - | - | - | - | - |
| | | Dioctyltin dilaurate | - | - | - | - | - | - |
| Average number of hydroxyl groups per molecule of oxyalkylene polymer A | | | 2.5 | 2.5 | 2.5 | 2.5 | 2.5 | 2.5 |
| Average number of hydroxyl groups per molecule of oxyalkylene polymer | | | 2.1 | 2.1 | 2.1 | 2.1 | 2.1 | 2.1 |
| Isocyanate index | | | 85 | 85 | 85 | 85 | 85 | 85 |
| Average content of EO units in hydroxyl-terminated urethane prepolymer [% by mass] | | | 16.8 | 13.0 | 10.0 | 8.8 | 7.0 | 5.0 |
| Average content of EO units in oxyalkylene polymer [% by mass] | | | 16.8 | 13.0 | 10.0 | 8.8 | 7.0 | 5.0 |
| Hydroxyl-terminated urethane prepolymer | | | U1 | U2 | U3 | U4 | U5 | U6 |

Table 2 (continued)

| | | | Synthesis example 2-7 | Synthesis example 2-8 | Synthesis example 2-9 | Synthesis example 2-10 | Synthesis example 2-11 |
|---|---|---|---|---|---|---|---|
| Amount blended [part by mass] | Oxyalkylene polymer | Polymer A1-1 | - | - | 30 | 36 | 36 |
| | | Polymer Al-2 | 36 | 30 | - | - | - |
| | | Polymer A2-1 | - | - | 70 | 48 | 48 |
| | | Polymer A2-2 | 48 | 70 | - | - | - |
| | | Polymer B1 | 16 | - | - | 16 | 16 |
| | Diisocyanate compound | 50M-HDI | 1.7 | 1.7 | 1.7 | 1.7 | 1.7 |
| | Catalyst | XK627 | 0.038 | 0.038 | 0.038 | - | - |
| | | Dibutyltin dilaurate | - | - | - | 0.008 | - |
| | | Dioctyltin dilaurate | - | - | - | - | 0.008 |
| Average number of hydroxyl groups per molecule of oxyalkylene polymer A | | | 2.5 | 2.3 | 2.3 | 2.5 | 2.5 |
| Average number of hydroxyl groups per molecule of oxyalkylene polymer | | | 2.1 | 2.3 | 2.3 | 2.1 | 2.1 |
| Isocyanate index | | | 85 | 85 | 85 | 85 | 85 |
| Average content of EO units in hydroxyl-terminated urethane prepolymer [% by mass] | | | 0.0 | 0.0 | 20.0 | 16.8 | 16.8 |
| Average content of EO units in oxyalkylene polymer [% by mass] | | | 0.0 | 0.0 | 20.0 | 16.8 | 16.8 |
| Hydroxyl-terminated urethane prepolymer | | | U7 | U8 | U9 | U10 | U11 |

[Production of Patch]

(Example 1)

A liquid (adhesive composition) obtained by mixing 100 parts by mass of 50% by mass solution of hydroxyl-terminated urethane prepolymer U1 and 5 parts by mass of a curing agent (E402-80B) were mixed and deaerated, and then the resulting product was coated on a polyester film (thickness of 38 $\mu$m) as the release liner with a knife coater and dried at 100°C for 2 minutes to form an adhesive agent layer of 25 $\mu$m thickness. On the adhesive agent layer, a polyester film (thickness of 75 $\mu$m) was adhered as a substrate and cured in a hot air dryer at 40°C for 3 days to produce a patch.

(Examples 2 to 11)

Patches were produced in the same manner as in example 1, except that the hydroxyl-terminated urethane prepolymer U1 in example 1 was changed to each of the hydroxyl-terminated urethane prepolymers U2 to U11.

(Examples 12 to 14)

Patches were produced in the same manner as in example 1, except that the adhesive composition obtained by mixing the hydroxyl-terminated urethane prepolymer U1 and the curing agent in example 1 was changed to the following commercially available products.

Example 12: commercially available acrylic adhesive agent (1525L; manufactured by 3M Company)

Example 13: commercially available silicone adhesive agent (Micropore S2552; manufactured by 3M Company)

Example 14: commercially available silicone adhesive agent (2476P; manufactured by 3M Company)

[Evaluation of Patch]

The following various evaluations were performed for the patches produced in examples 1 to 14. The evaluation results are shown in Tables 3 and 4. Note that examples 1 to 4 are Examples, and examples 5 to 14 are Comparative Examples.

<Adhesive Force>

(Versus Phenol Resin)

The patch (100 mm high and 15 mm wide) from which the release liner was peeled off was overlapped on the side of the adhesive agent layer with a phenol resin plate ("Sumilite (registered trademark) PL-1102", manufactured by Sumitomo Bakelite Co., Ltd.; 125 mm high, 30 mm wide, 2 mm thick), and a rubber roller was rolled over thereon from the side of the substrate of the patch with a load of 2 kg and a rate of 300 mm/minute for one return to compression bond the adhesive agent layer to the phenol resin plate.
After leaving still for 20 minutes, the adhesive force when the adherend was a phenol resin (versus phenol resin) was measured by peeling off the patch at a peeling angle of 90° and a rate of 300 mm/minute by a method in accordance with JIS Z 0237:2009.
Table 3 shows the average value of three measurement values. Also, the measured adhesive force was evaluated based on the following evaluation criteria (phenol resin).

A...3.0 (N/15 mm) or more
B...2.5 (N/15 mm) or more and less than 3.0 (N/15 mm)
C...2.0 (N/15 mm) or more and less than 2.5 (N/15 mm)
D...less than 2.0 (N/15 mm)

When evaluated as evaluation A and B, it can be said that the adhesive force to the phenol resin is sufficient, which is a representative material for the sealant of the circuit pattern for a wearable device, or the like. On the other hand, when evaluated as evaluations C and D, the adhesive force to the phenol resin was insufficient.

(Versus Skin)

The adhesive force was measured for the case where the adherend was the skin of the forearm of humans (3 subjects) (versus skin), in the same manner as in the case where the adherend was the phenol resin.
Table 3 shows the average value of measurement values. Also, the measured adhesive force was evaluated based on the following evaluation criteria (versus skin).

A+: 1.0 N/15 mm or more and less than 2.7 N/15 mm
A: 2.7 N/15 mm or more and less than 3.0 N/15 mm
B: less than 1.0 N/15 mm
C: 3.0 N/15 mm or more

When evaluated as evaluation A, it can be said that the adhesive force to the skin is adequate and skin friendly. When evaluated as evaluation A+, it can be said that pain to the skin is less occurred, and the adhesive force to the skin is rather adequate. On the other hand, when evaluated as evaluation B, the adhesive force to the skin is too small so that it is easy to be peeled off. Further, when evaluated as evaluation C, the adhesive force is too strong to peel off from the skin, and it may cause pain when peeling off.

(Difference)

The value of the adhesive force to the skin was subtracted from the value of the adhesive force to the phenol resin measured above to calculate the difference in the adhesive force between to the phenol resin and to the skin.
The difference in the adhesive force between to the phenol resin and to the skin was evaluated based on the following evaluation criteria.

A: 2.5 N/15 mm or more
B: 1.8 N/15 mm or more and less than 2.5 N/15 mm
C: 1.5 N/15 mm or more and less than 1.8 N/15 mm
D: less than 1.5 N/15 mm

When evaluated as evaluation A and B, it can be said that the wearable device can be gently attached to and detached from the skin. On the other hand, when evaluated as evaluations C and D, it is difficult to stably attach the wearable device to the skin.

(Skin Observation)

A specimen (20 mm high and 15 mm wide) of the patch was attached to the skin of forearm of humans (3 subjects) in an atmosphere of 23°C and 50% RH. After one hour, the specimen was peeled off from the skin, and the skin condition was visually observed.
The observation results were evaluated based on the following evaluation criteria.

A: No change from before the specimen being attached.
B: Some subjects had redness on the skin.
C: Some subjects had adhesive residues (residues of adhesive agent).

When evaluated as evaluation A, it can be said that it is skin friendly. On the other hand, when evaluated as evaluation B or C, it is determined to be not skin friendly.

(Cytotoxicity Test)

For the adhesive agent layer collected from the patches, the colony formation rates were determined by conducting a cytotoxicity test (cell line: V79 cells, medium: MEM10 medium) using a colony formation method by an extract method, in accordance with ISO 10993-5:2009 "Biological evaluation of medical devices -- Part 5: Tests for in vitro cytotoxicity".
The test liquid used was an extract obtained by extracting 1 g of the adhesive agent layer with a medium at 37°C for 24 hours.
The colony formation rates were evaluated based on the following evaluation criteria.

A: 70% or more
B: 50% or more and less than 70%
C: less than 50%

When evaluated as evaluation A and B, it can be said that there is no or very little cytotoxicity, and therefore it is skin friendly. On the other hand, when evaluated as evaluation C, the cytotoxicity is determined to be stronger.
Note that the symbol "-" in the columns of the cytotoxicity test in Table 3 indicates that the test was not conducted.

(Antibacterial Test)

An antibacterial test was conducted on the adhesive agent layer collected from the patches using Staphylococcus aureus and Escherichia coli in accordance with Japanese Industrial Standards JIS Z 2801:2010 (Antimicrobial products - Test for antimicrobial activity and efficacy) and ISO22196:2011, Measurement of antibacterial activity on plastics and other non-porous surfaces.
To specimens (antibacterial processed products and non-processed products) of 50 mm × 50 mm (thickness of 10 mm or less), 0.4 mL of bacterial solution was added dropwise, and the specimens were covered with a film of 40 mm × 40 mm. The specimens were cultured at 35 ± 1°C and a relative humidity of 90% or more for 24 hours. After leaving still, test bacteria on the specimens were washed away and collected, and then the viable cell counts per $cm^2$ were collected. The antibacterial activity values were calculated by the following formula.

$$R = Ut - At$$

R: Antibacterial activity value
Ut: Average value of logarithm values of the viable cell counts per $cm^2$ after 24 hours on non-processed specimens
At: Average value of logarithm values of the viable cell counts per $cm^2$ after 24 hours on antibacterial processed specimens

The antibacterial properties were evaluated based on the following evaluation criteria.

A: Both the antibacterial activity values of Staphylococcus aureus and Escherichia coli were 2.0 or more.
B: One of the antibacterial activity values of Staphylococcus aureus and Escherichia coli was 2.0 or more, and the other was less than 2.0.
C: Both the antibacterial activity values of Staphylococcus aureus and Escherichia coli were less than 2.0.

When evaluated as evaluation A, it can be said that it is antibacterial. On the other hand, when evaluated as evaluation B or C, it is determined to be not antibacterial.
Note that the symbol "-" in the columns of the antibacterial test in Table 3 indicates that the test was not conducted.

[Table 3]

Table 3

| | | | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|---|---|
| Amount blended [part by mass] | Hydroxyl-terminated urethane prepolymer | Urethane prepolymer U1 | 100 | - | - | - | - | - | - | - | - | - | - |
| | | Urethane prepolymer U2 | - | 100 | - | - | - | - | - | - | - | - | - |
| | | Urethane prepolymer U3 | - | - | 100 | - | - | - | - | - | - | - | - |
| | | Urethane prepolymer U4 | - | - | - | 100 | - | - | - | - | - | - | - |
| | | Urethane prepolymer U5 | - | - | - | - | 100 | - | - | - | - | - | - |
| | | Urethane prepolymer U6 | - | - | - | - | - | 100 | - | - | - | - | - |
| | | Urethane prepolymer U7 | - | - | - | - | - | - | 100 | - | - | - | - |
| | | Urethane prepolymer U8 | - | - | - | - | - | - | - | 100 | - | - | - |
| | | Urethane prepolymer U9 | - | - | - | - | - | - | - | - | 100 | - | - |
| | | Urethane prepolymer U10 | - | - | - | - | - | - | - | - | - | 100 | - |
| | | Urethane prepolymer U11 | - | - | - | - | - | - | - | - | - | - | 100 |
| | Curing agent | Duranate E402-80B | 5 | 5 | 5 | 5 | 5 | 5 | 5 | 5 | 5 | 5 | 5 |
| Film thickness of adhesive agent [μm] | | | 25 | 25 | 25 | 25 | 25 | 25 | 25 | 25 | 25 | 25 | 25 |
| Characteristics | Adhesive force | Versus phenol resin [N/15mm] | 6.0 | 5.8 | 5.7 | 5.5 | 5.0 | 4.9 | 4.5 | 3.1 | 4.2 | 4.1 | 2.7 |
| | | Versus phenol resin Evaluation | A | B | A | A | A | A | A | A | A | A | B |
| | | Versus skin [N/15mm] | 2.9 | 2.6 | 2.7 | 2.7 | 3.2 | 3.3 | 3.0 | Unmeasurable | Unmeasurable | 1.5 | 1.1 |
| | | Versus skin Evaluation | A | A+ | A+ | A+ | C | C | C | B | B | A+ | A+ |
| | | Difference [N/15mm] | 3.1 | 3.2 | 3.0 | 2.8 | 1.8 | 1.6 | 1.5 | Not attached to skin | Not attached to skin | 2.6 | 1.6 |
| | | Difference Evaluation | A | A | A | A | c | C | C | - | - | A | C |
| | Skin observation | | A | A | A | A | B | B | B | - | - | A | C |

(continued)

| | | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|---|
| Antibacterial test | Antibacterial activity against Staphylococcus aureus | 2.7 | 2.5 | 2.3 | 3.3 | 1.3 | 1.2 | 0 | 0 | 2.7 | 3.8 | - |
| | Antibacterial activity against Escherichia coli | 4.7 | 4 | 3.5 | 3.6 | 0.3 | 2.1 | 0.9 | 0.8 | 4.8 | 3.2 | - |
| | Evaluation | A | A | A | A | c | B | C | C | A | A | - |
| Cytotoxicity test | Colony formation rate [%] | 91.0 | - | - | 90.8 | - | - | - | - | - | 0 | 36.8 |
| | Evaluation | A | - | - | A | - | - | - | - | - | C | B |

[Table 4]

[Table 4]

Table 4

| | | | Example 1 | Example 12 | Example 13 | Example 14 |
|---|---|---|---|---|---|---|
| Amount blended [part by mass] | Hydroxyl-terminated urethane prepolymer | Urethane prepolymer U1 | 100 | Commercially available acrylic adhesive agent | Commercially available silicone adhesive agent | Commercially available silicone adhesive agent |
| | Curing agent | Duranate E402-80B | 5 | | | |
| Characteristics | Adhesive force | Versus phenol resin — [N/15mm] | 6.0 | 6.4 | 2.0 | 2.3 |
| | | Versus phenol resin — Evaluation | A | A | C | C |
| | | Versus skin — [N/15mm] | 2.9 | 4.0 | 1.1 | 1.2 |
| | | Versus skin — Evaluation | A | C | A+ | A+ |
| | | Difference — [N/15mm] | 3.1 | 2.4 | 0.9 | 1.1 |
| | | Difference — Evaluation | A | B | D | D |
| | Skin observation | | A | B | A | A |
| | Antibacterial test | Antibacterial activity value against Staphylococcus aureus | 2.7 | 5.5 | 0 | 0 |
| | | Antibacterial activity value against Escherichia coli | 4.7 | 5.0 | 0 | 0 |
| | | Evaluation | A | A | C | C |

[Evaluation Results]

In Table 3, examples 1 to 4 correspond to Examples of the present invention, and examples 5 to 11 correspond to Comparative Examples of the present invention.

When the evaluation of the adhesive force (versus phenol resin) is A or B, the evaluation of the adhesive force (versus skin) is A+ or A, and the evaluation of the difference in the adhesive force is A or B, low adhesion to the skin and high adhesion to a substrate are both achieved. In addition, when the evaluation of the skin observation is A, the adhesive agent can be peeled off without leaving on the skin, and therefore, it can be said that the adhesive agent is skin friendly. When the evaluation of the cytotoxicity test is A or B, it can be said that the material is guaranteed to prevent damage to the skin when attached to the skin and to be safe when attached for a long time. When the evaluation of the antibacterial test is A, it can be said that the material is guaranteed to have antibacterial properties when attached to the skin, and it can be said to be safe for skin when attached for a long time.

The adhesive agents of examples 1 to 4 can achieve both the low adhesion to the skin and high adhesion to the substrate and can be peeled off without leaving the adhesive agent on the skin, are skin friendly, guaranteed to prevent damage to the skin when applied to the skin, to be safe when applied for a long time, and to be antibacterial when applied to the skin.

In contrast, the results of the adhesive agents of examples 5 to 7 were inferior in the evaluation of their antibacterial test as well as their adhesive force. This is thought to be caused by the decreased average content of EO units in the oxyalkylene polymer A used in the synthesis of the hydroxyl-terminated urethane prepolymer.

The adhesive agents of examples 8 and 9 are examples in which the oxyalkylene polymer B (monofunctional polyol) is not used, no adhesive force can be exerted without using the oxyalkylene polymer B, and therefore, these adhesive agents cannot be used as a patch.

The adhesive agents of examples 10 and 11 were inferior in the evaluation of the cytotoxicity test. This is thought to be caused by the use of a tin-based catalyst, which is considered highly toxic in the synthesis of a urethane prepolymer.

Table 4 shows the results of the comparison of commercially available patches (for medical use) and the patch of the present invention. The commercially available patches of examples 12 to 14 (for medical use) did not have antibacterial properties or did not achieve both the low adhesion to the skin and the high adhesion to the substrate.

**Claims**

1.  An adhesive composition comprising a hydroxyl-terminated urethane prepolymer and a polyisocyanate compound, wherein

    the hydroxyl-terminated urethane prepolymer is a reaction product of an oxyalkylene polymer and a diisocyanate compound in the presence of a tin-free catalyst,
    the oxyalkylene polymer comprises an oxyalkylene polymer A having the number of hydroxyl groups per molecule of 2 or more and an oxyalkylene polymer B having the number of hydroxyl groups per molecule of 1, and
    the hydroxyl-terminated urethane prepolymer has an average content of structural units based on ethylene oxide of 8 to 50% by mass.

2.  The adhesive composition according to claim 1, wherein the content of the tin-free catalyst is more than 0.001 parts by mass and less than 0.050 parts by mass per 100 parts by mass of the oxyalkylene polymer.

3.  The adhesive composition according to claim 1 or 2, wherein the tin-free catalyst is an organometallic catalyst.

4.  The adhesive composition according to claim 1 or 2, wherein the tin-free catalyst comprises one or more metals selected from the group consisting of zinc and bismuth.

5.  The adhesive composition according to claim 1 or 2, wherein the oxyalkylene polymer has an average content of structural units based on ethylene oxide of 8 to 50% by mass.

6.  The adhesive composition according to claim 1 or 2, wherein the oxyalkylene polymer has an average number of hydroxyl groups per molecule of 1.5 to 3.0.

7.  The adhesive composition according to claim 1 or 2, wherein the oxyalkylene polymer A comprises an oxyalkylene polymer A1 having the number of hydroxyl groups per molecule of 3 and an oxyalkylene polymer A2 having the number of hydroxyl groups per molecule of 2.

8. The adhesive composition according to claim 1 or 2, wherein the oxyalkylene polymer A has an average content of structural units based on ethylene oxide of 0 to 80% by mass.

9. The adhesive composition according to claim 1 or 2, wherein the number-average molecular weight of the oxyalkylene polymer B is 4000 to 30000.

10. The adhesive composition according to claim 1 or 2, used for forming an adhesive agent that comes into contact with skin.

11. An adhesive composition comprising a hydroxyl-terminated urethane prepolymer, a polyisocyanate compound, and a tin-free catalyst, wherein
the hydroxyl-terminated urethane prepolymer has an average content of structural units based on ethylene oxide of 8 to 50% by mass.

12. A method for producing an adhesive composition by reacting an oxyalkylene polymer and a diisocyanate compound in the presence of a tin-free catalyst to obtain a hydroxyl-terminated urethane prepolymer, and

mixing the hydroxyl-terminated urethane prepolymer and a polyisocyanate compound, wherein
the oxyalkylene polymer comprises an oxyalkylene polymer A having the number of hydroxyl groups per molecule of 2 or more and an oxyalkylene polymer B having the number of hydroxyl groups per molecule of 1, and
the hydroxyl-terminated urethane prepolymer has an average content of structural units based on ethylene oxide of 8 to 50% by mass.

13. An adhesive agent, being a cured product of the adhesive composition according to claim 1.

14. The adhesive agent according to claim 13, which passes the antibacterial test standard described in ISO22196:2011 or JIS Z2801:2010.

15. A patch comprising a substrate and an adhesive agent layer provided on at least a part of a surface of the substrate, wherein the adhesive agent layer comprises the adhesive agent according to claim 13.

16. The patch according to claim 15, being an adhesive tape.

17.

A wearable device comprising a wearable device main body and an adhesive agent layer, wherein
the adhesive agent layer is provided on at least a part of the surface of the wearable device facing the skin and comprises the adhesive agent according to claim 13.

18. A wearable device kit comprising a wearable device main body, and at least any of the adhesive agent according to claim 13 and the patch according to claim 15.

## INTERNATIONAL SEARCH REPORT

| | International application No. |
| --- | --- |
| | **PCT/JP2024/011799** |

**A. CLASSIFICATION OF SUBJECT MATTER**

*C09J 175/08*(2006.01)i; *C09J 7/38*(2018.01)i; *C09J 11/06*(2006.01)i; *A61F 13/02*(2024.01)n; *A61F 13/0246*(2024.01)n; *C08G 18/10*(2006.01)n; *C08G 18/48*(2006.01)n

FI: C09J175/08; C09J7/38; C09J11/06; A61F13/02 Z; A61F13/0246 501; C08G18/48 033; C08G18/48 037; C08G18/10

According to International Patent Classification (IPC) or to both national classification and IPC

**B. FIELDS SEARCHED**

Minimum documentation searched (classification system followed by classification symbols)

C09J1/00-201/10; A61F13/02; A61F13/0246; C08G18/00-18/87,71/00-71/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2020/175220 A1 (AGC INC.) 03 September 2020 (2020-09-03) claims 1, 7, 17-20, paragraphs [0009], [0019], [0038], [0050], [0112]-[0114], [0124], tables 1-3, examples 2-5 | 1-18 |
| Y | WO 2017/043173 A1 (MITSUI CHEMICALS TOHCELLO, INC.) 16 March 2017 (2017-03-16) claim 1, paragraphs [0002], [0105], table 1, examples 1-5 | 1-18 |
| Y | US 2019/0322091 A1 (PLASTICS INDUSTRY DEVELOPMENT CENTER) 24 October 2019 (2019-10-24) claim 1, paragraph [0045], fig. 1 | 1-18 |
| Y | JP 6703945 B2 (DOW GLOBAL TECHNOLOGIES LLC) 03 June 2020 (2020-06-03) claims 1, 3, paragraph [0022] | 1-18 |
| A | JP 2019-214655 A (TOSOH CORP.) 19 December 2019 (2019-12-19) claims 1, 13-14, tables 1-5, examples 1-14 | 1-18 |

☐ Further documents are listed in the continuation of Box C.      ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **06 June 2024** | **18 June 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT
### Information on patent family members

International application No.

**PCT/JP2024/011799**

| Patent document cited in search report | | | Publication date (day/month/year) | Patent family member(s) | | | Publication date (day/month/year) |
|---|---|---|---|---|---|---|---|
| WO | 2020/175220 | A1 | 03 September 2020 | US | 2021/0371574 | A1 | |
| | | | | claims 1, 7, 17-22, paragraphs [0056], [0075], [0096], [0120]-[0127], [0239]-[0242], [0282]-[0284], tables 1-3, examples 2-5 | | | |
| | | | | EP | 3933006 | A1 | |
| | | | | CN | 113518813 | A | |
| WO | 2017/043173 | A1 | 16 March 2017 | US | 2018/0237672 | A1 | |
| | | | | claim 1, paragraphs [0002], [0160], table 1, examples 1-5 | | | |
| | | | | EP | 3348623 | A1 | |
| | | | | CN | 107949614 | A | |
| | | | | KR | 10-2018-0050717 | A | |
| | | | | TW | 201710453 | A | |
| US | 2019/0322091 | A1 | 24 October 2019 | TW | 201943429 | A | |
| | | | | CN | 110384819 | A | |
| JP | 6703945 | B2 | 03 June 2020 | US | 2016/0208040 | A1 | |
| | | | | claim 1, paragraph [0022] | | | |
| | | | | WO | 2015/038827 | A1 | |
| | | | | CN | 105518047 | A | |
| | | | | EP | 3044244 | A1 | |
| JP | 2019-214655 | A | 19 December 2019 | (Family: none) | | | |

Form PCT/ISA/210 (patent family annex) (July 2022)

**EP 4 692 268 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022098300 A
- JP 2022541292 A
- WO 2003062301 A
- WO 2004067633 A
- JP 2004269776 A
- JP 2005015786 A
- WO 2013065802 A
- JP 2015010162 A